# EUROPEAN PATENT APPLICATION

(11) **EP 4 353 726 A1**
(43) Date of publication of application: **17.04.2024**
(21) Application number: 22837544.0
(22) Date of filing: 28.06.2022
(51) Int. Cl.: C07D 487/04, C07D 241/36

(54) **HOLE TRANSPORT PROMOTING MATERIAL, MATERIAL FOR LIGHT RECEIVING ELEMENT, CYANO COMPOUND, AND ORGANIC LIGHT RECEIVING ELEMENT**

(30) Priority: 07.07.2021 JP 2021112797
(71) Applicant: Tosoh Corporation, Yamaguchi 746-8501 (JP)
(72) Inventor: ARAI Nobumichi, Ayase-Shi, Kanagawa 252-1123 (JP); OKADA Takeshi, Ayase-Shi, Kanagawa 252-1123 (JP); TAKAHASHI Yasuhiro, Ayase-Shi, Kanagawa 252-1123 (JP); NOMURA Keisuke, Tokyo 105-8623 (JP)
(74) Representative: Novagraaf International SA
(86) International application number: PCT/JP2022/025802
(87) International publication number: WO 2023/282128

(57) **Abstract**

Provided are a material for a light receiving element having excellent hole transport characteristics, and an organic light receiving element using the same.

A material for a light receiving element is used that includes a compound represented by general formula (1) or general formula (2). Ar² and Ar³ are preferably cyano groups. Ar⁴ and Ar⁵ are preferably cyano groups.

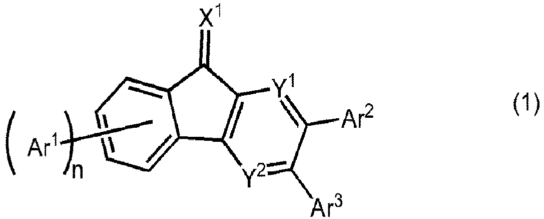

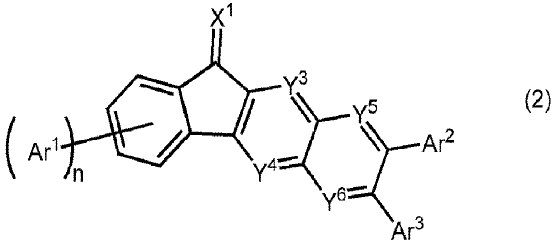

## Description

### TECHNICAL FIELD

The present invention relates to a hole transport promoting material, a material for a light receiving element, a cyano compound, and an organic light receiving element.

### BACKGROUND ART

Light receiving elements are elements having a function of converting received light into an electric signal or electric energy. Among the light receiving elements, a light receiving element for imaging element is used in applications such as a mobile phone and a camera, and thus, development of the light receiving element is actively performed.

In recent years, demand for the light receiving element for imaging element has been increasing from the market, and a material having excellent characteristics in all of charge transport characteristics, dark current, quantum efficiency, and the like has been required. As an organic light receiving element having a light receiving layer made of an organic material, a laminated structure is common, which has a charge transporting layer such as a hole transporting layer that transports holes generated in the light receiving layer to a first electrode and an electron transporting layer that transports electrons generated in the light receiving layer to a second electrode (for example, refer to Patent Document 1). However, even when such a charge transporting layer is provided, sufficient charge transport characteristics cannot be said to be obtained, and an organic light receiving element excellent in charge transport characteristics is required. Further, when a charge transporting layer having high charge transport characteristics is used, dark current characteristics of the organic light receiving element easily deteriorate. It is, thus, required to simultaneously enhance the two characteristics.

### Citation List

### Patent Document

Patent Document 1: PCT International Publication No. WO2015/163349

### DISCLOSURE OF THE INVENTION

### Problems to be Solved by the Invention

The present invention has been made in view of the above circumstances, and an object of the present invention is to provide a material for a light receiving element excellent in hole transport characteristics, and an organic light receiving element using the same.

### Means for Solving the Problems

The present inventors have found that the above problem can be solved by providing a layer containing a specific cyano compound between a hole transporting layer responsible for hole transport and an electrode to which holes are transported, thereby arriving at the completion of the present invention.

Embodiments of the present invention relate to the following hole transport promoting material, a material for light receiving element, a cyano compound, and an organic light receiving element.

A first aspect of the present invention relates to a hole transport promoting material including a compound represented by the following general formula (1) or (2): 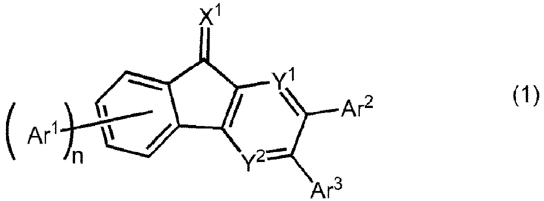 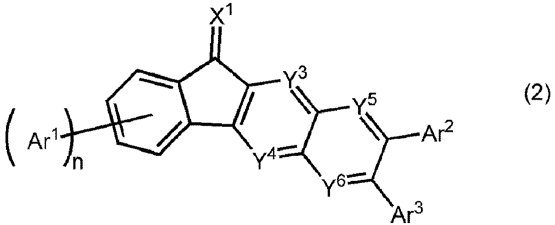 wherein
Y¹, Y², Y³, Y⁴, Y⁵ and Y⁶ each independently represent a nitrogen atom or C-H;
at least one of Y¹ or Y² is a nitrogen atom;
at least one of Y³, Y⁴, Y⁵ or Y⁶ is a nitrogen atom;
Ar¹, Ar² and Ar³ each independently represent:
   a hydrogen atom,
   a cyano group,
   a fluoro group,
   a fluoroalkyl group,
   an aryl group having 6 to 18 carbon atoms and being optionally substituted with a fluoro group, a fluoroalkyl group, or a cyano group; or
   a heteroaryl group having 3 to 17 carbon atoms and being optionally substituted with a fluoro group, a fluoroalkyl group, or a cyano group;
   n represents an integer of 1 to 4; and
   X¹ is a divalent group represented by the following formula (a) or (b): 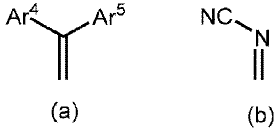 wherein,
      Ar⁴ and Ar⁵ each independently represent:
      a cyano group,
      a fluoro group,
      a fluoroalkyl group,
      an aryl group having 6 to 18 carbon atoms and being optionally substituted with a fluoro group, a fluoroalkyl group, or a cyano group; or
      a heteroaryl group having 3 to 17 carbon atoms and being optionally substituted with a fluoro group, a fluoroalkyl group, or a cyano group.

A second aspect of the present invention relates to the hole transport promoting material as described in the first aspect, in which Ar² and Ar³ are cyano groups.

A third aspect of the present invention relates to the hole transport promoting material as described in the first or second aspect, in which Ar⁴ and Ar⁵ are cyano groups.

A fourth aspect of the present invention relates to a light receiving element material including a compound represented by the following general formula (1) or (2): 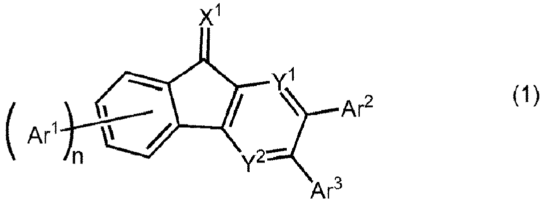 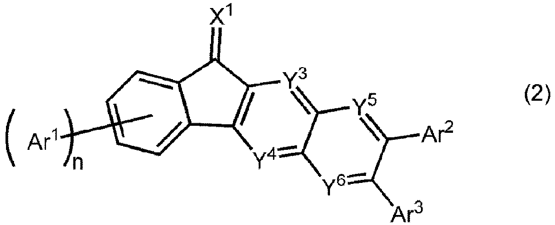 wherein
Y¹, Y², Y³, Y⁴, Y⁵ and Y⁶ each independently represent a nitrogen atom or C-H;
at least one of Y¹ or Y² is a nitrogen atom;
at least one of Y³, Y⁴, Y⁵ or Y⁶ is a nitrogen atom;
Ar¹, Ar² and Ar³ each independently represent:
   a hydrogen atom,
   a cyano group,
   a fluoro group,
   a fluoroalkyl group,
   an aryl group having 6 to 18 carbon atoms and being optionally substituted with a fluoro group, a fluoroalkyl group, or a cyano group; or
   a heteroaryl group having 3 to 17 carbon atoms and being optionally substituted with a fluoro group, a fluoroalkyl group, or a cyano group;
   n represents an integer of 1 to 4; and
   X¹ is a divalent group represented by the following formula (a) or (b): 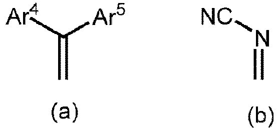 wherein
      Ar⁴ and Ar⁵ each independently represent:
      a cyano group,
      a fluoro group,
      a fluoroalkyl group,
      an aryl group having 6 to 18 carbon atoms and being optionally substituted with a fluoro group, a fluoroalkyl group, or a cyano group; or
      a heteroaryl group having 3 to 17 carbon atoms and being optionally substituted with a fluoro group, a fluoroalkyl group, or a cyano group.

A fifth aspect of the present invention relates to the light receiving element material as described in the fourth aspect, in which the light receiving element material is an organic imaging element material for light receiving element.

A sixth aspect of the present invention relates to the light receiving element material as described in the fourth or fifth aspect, in which the light receiving element material is a hole transport promoting material for organic imaging element for light receiving element.

A seventh aspect of the present invention relates to the light receiving element material as described in any one of the fourth to sixth aspects, in which Ar² and Ar³ are cyano groups.

An eighth aspect of the present invention relates to the light receiving element material as described in any one of the fourth to seventh aspects, in which Ar⁴ and Ar⁵ are cyano groups.

A ninth aspect of the present invention relates to a cyano compound represented by the following general formula (3), general formula (4), general formula (5) or general formula (6) : 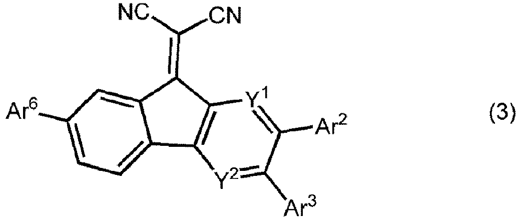 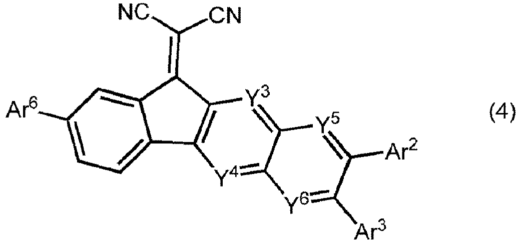 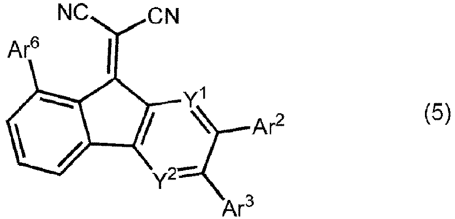 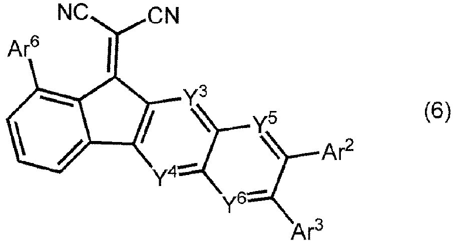 wherein
Y¹, Y², Y³, Y⁴, Y⁵ and Y⁶ each independently represent a nitrogen atom or C-H;
at least one of Y¹ or Y² is a nitrogen atom;
at least one of Y³, Y⁴, Y⁵, or Y⁶ is a nitrogen atom;
Ar⁶ represents:
   a cyano group,
   A fluoro group, a chloro group, a bromo group, an iodine group,
   a fluoroalkyl group,
   an aryl group having 6 to 18 carbon atoms and being optionally substituted with a fluoro group, a fluoroalkyl group, or a cyano group; or
   a heteroaryl group having 3 to 17 carbon atoms and being optionally substituted with a fluoro group, a fluoroalkyl group, or a cyano group;
   provided that when Y⁵ and Y⁶ are C-H, Ar⁶ may be a hydrogen atom, and
   Ar² and Ar³ each independently represent:
      a hydrogen atom,
      a cyano group,
      a fluoro group,
      a fluoroalkyl group,
      an aryl group having 6 to 18 carbon atoms and being optionally substituted with a fluoro group, a fluoroalkyl group, or a cyano group; or
      a heteroaryl group having 3 to 17 carbon atoms and being optionally substituted with a fluoro group, a fluoroalkyl group, or a cyano group.

A tenth aspect of the present invention relates to the cyano compound as described in the ninth aspect, in which Ar² and Ar³ are cyano groups.

An eleventh aspect of the present invention relates to an organic light receiving element, including a first electrode and a second electrode, and
having an organic layer between the first electrode and the second electrode,
the organic layer including at least a light receiving layer, a hole transporting layer, and a hole transport promoting layer,
the hole transport promoting layer including a compound having a partial structure represented by the following formula (Q): 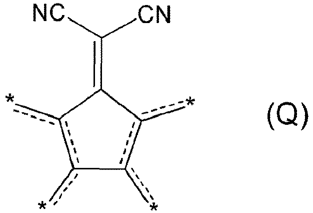 wherein portions in which solid lines and broken lines are parallel each independently represent a single bond or a double bond, and * represents a bond.

A twelfth aspect of the present invention relates to an organic light receiving element, including a first electrode and a second electrode, and
having an organic layer between the first electrode and the second electrode,
the organic layer including at least a light receiving layer, a hole transporting layer, and a hole transport promoting layer,
the hole transport promoting layer including a compound having a partial structure represented by the following formula (1) or (2): 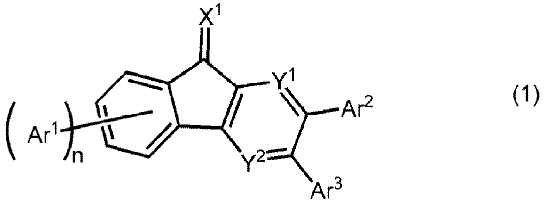 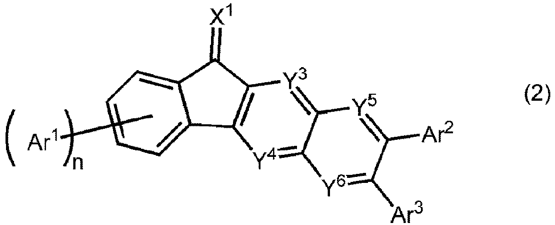 wherein
   Y¹, Y², Y³, Y⁴, Y⁵ and Y⁶ each independently represent a nitrogen atom or C-H;
   at least one of Y¹ or Y² is a nitrogen atom;
   at least one of Y³, Y⁴, Y⁵ or Y⁶ is a nitrogen atom;
   Ar¹, Ar² and Ar³ each independently represent:
      a hydrogen atom,
      a cyano group,
      a fluoro group,
      a fluoroalkyl group,
      an aryl group having 6 to 18 carbon atoms and being optionally substituted with a fluoro group, a fluoroalkyl group, or a cyano group; or
      a heteroaryl group having 3 to 17 carbon atoms and being optionally substituted with a fluoro group, a fluoroalkyl group, or a cyano group,
      n represents an integer of 1 to 4; and
      X¹ is a divalent group represented by the following formula (a) or (b): 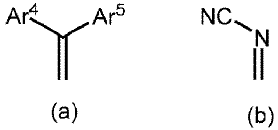 wherein
         Ar⁴ and Ar⁵ each independently represent:
         a hydrogen atom,
         a fluoro group,
         a fluoroalkyl group,
         an aryl group having 6 to 18 carbon atoms and being optionally substituted with a fluoro group, a fluoroalkyl group, or a cyano group; or
         a heteroaryl group having 3 to 17 carbon atoms and being optionally substituted with a fluoro group, a fluoroalkyl group, or a cyano group.

A thirteenth aspect of the present invention relates to the organic light receiving element as described in the eleventh or twelfth aspect, in which the hole transporting layer and the hole transport promoting layer are adjacent to each other.

A fourteenth aspect of the present invention relates to the organic light receiving element as described in any one of the eleventh to thirteenth aspects, in which the light receiving layer is a layer composed of at least two components.

### Effects of the Invention

According to the present invention, it is possible to provide a light receiving element material excellent in hole transport characteristics and an organic light receiving element using the same.

### BRIEF DESCRIPTION OF THE DRAWINGS

FIG. 1 is a schematic cross-sectional view showing an example of an organic light receiving element according to an embodiment of the present invention; and
FIG. 2 is a diagram showing evaluation results of film quality of the hole transport promoting layers in Reference Example 1 and Comparative Reference Example 1.

### PREFERRED MODE FOR CARRYING OUT THE INVENTION

### <Light Receiving Element Material>

The light receiving element material of the present embodiment includes a compound represented by the following general formula (1) or (2). In this specification, the term "material" includes compounds.

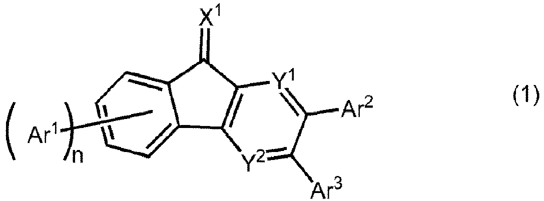

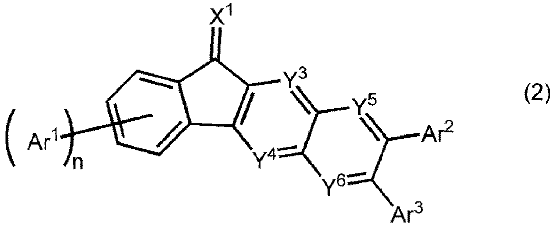

In the formulae (1) and (2),
Y¹, Y², Y³, Y⁴, Y⁵ and Y⁶ each independently represent a nitrogen atom or C-H;
at least one of Y¹ or Y² is a nitrogen atom;
at least one of Y³, Y⁴, Y⁵ or Y⁶ is a nitrogen atom;
Ar¹, Ar² and Ar³ each independently represent:
   a hydrogen atom,
   a cyano group,
   a fluoro group,
   a fluoroalkyl group,
   an aryl group having 6 to 18 carbon atoms and being optionally substituted with a fluoro group, a fluoroalkyl group, or a cyano group; or
   a heteroaryl group having 3 to 17 carbon atoms and being optionally substituted with a fluoro group, a fluoroalkyl group, or a cyano group;
   n represents an integer of 1 to 4; and
   X¹ is a divalent group represented by the following formula (a) or (b): 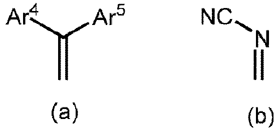 in the formula (a),
      Ar⁴ and Ar⁵ each independently represent
      a cyano group,
      a fluoro group,
      a fluoroalkyl group,
      an aryl group having 6 to 18 carbon atoms and being optionally substituted with a fluoro group, a fluoroalkyl group, or a cyano group; or
      a heteroaryl group having 3 to 17 carbon atoms and being optionally substituted with a fluoro group, a fluoroalkyl group, or a cyano group.

By providing a layer containing a light receiving element material represented by the general formula (1) or (2) between a hole transporting layer for transporting holes and an electrode to which the holes are transported, the hole transport capability of the light receiving element can be significantly improved.

### [Compound Represented by General Formula (1)]

Preferred embodiments of the compound represented by the general formula (1) are as follows from the viewpoint that the compound can significantly improve the hole transport capability of the light receiving element.

Y¹ and Y² are each preferably a nitrogen atom.

Examples of the aryl group having 6 to 18 carbon atoms represented by Ar¹, Ar², Ar³, Ar⁴ and Ar⁵ include a phenyl group, a biphenylyl group, a terphenylyl group, a naphthyl group, a phenanthryl group, an anthryl group, a fluorenyl group, a dimethylfluorenyl group, a spirofluorenyl group, a pyrenyl group, a fluoranthenyl group, a triphenylenyl group, a tetracenyl group, and a chrysenyl group. Among these, a phenyl group, a biphenylyl group and a naphthyl group are preferable. Among the substituents of the aryl group having 6 to 18 carbon atoms, examples of the fluoroalkyl group include a trifluoromethyl group, a pentafluoroethyl group, a heptafluoropropyl group, a nonafluorobutyl group, a undecafluoropentyl group, and a tridecafluorohexyl group. Among these, a trifluoromethyl group is preferable.

Examples of the heteroaryl group having 3 to 17 carbon atoms represented by Ar¹, Ar², Ar³, Ar⁴ and Ar⁵ include a pyridyl group, a bipyridyl group, a terpyridyl group, a phenylpyridyl group, a diphenylpyridyl group, a pyridylphenyl group, a pyrazyl group, a phenylpyrazyl group, a pyrazylphenyl group, a pyrimidyl group, a phenylpyrimidyl group, a diphenylpyrimidyl group, a pyrimidylphenyl group, a triazyl group, a phenyltriazyl group, a diphenyltriazyl group, a diphenyltriazylphenyl group, a quinolyl group, a phenylquinolyl group, a quinolylphenyl group, an isoquinolyl group, a phenylisoquinolyl group, a quinolylphenyl group, an azaanthryl group, a diazaanthryl group, a triazaanthryl group, a tetraazaanthryl group, an azaphenanthryl group, a diazaphenanthryl group, a triazaphenanthryl group, a tetraazaphenanthryl group, an azapyrenyl group, a diazapyrenyl group, a triazapyrenyl group, a tetraazapyrenyl group, an azafluoranthenyl group, a diazafluoranthenyl group, a triazafluoranthenyl group, a tetraazafluoranthenyl group, an azatriphenylenyl group, a diazatriphenylenyl group, a triazatriphenylenyl group, a tetraazatriphenylenyl group, a pentaazatriphenylenyl group, and a hexaazatriphenylenyl group. Among these, a pyridyl group and a pyridylphenyl group are preferable. Among the substituents of the heteroaryl group having 3 to 17 carbon atoms, examples of the fluoroalkyl group include a trifluoromethyl group, a pentafluoroethyl group, a heptafluoropropyl group, a nonafluorobutyl group, a undecafluoropentyl group, and a tridecafluorohexyl group. Among these, a trifluoromethyl group is preferable.

Ar¹ is preferably a hydrogen atom, a fluoro group, a cyano group, a phenyl group or a pyridyl group. Ar² and Ar³ are preferably a cyano group, a trifluoromethyl group, a fluoro group, or a phenyl group. As X¹, a divalent group represented by the formula (a) is preferable. In the formula (a), as Ar⁴ and Ar⁵, a cyano group, a trifluoromethyl group, a pyridyl group and a phenyl group are preferable.

### [Compound represented by General Formula (2)]

Preferred embodiments of the compound represented by the general formula (2) are as follows because the compound can significantly improve the hole transport capability of the light receiving element.

Among Y³, Y⁴, Y⁵ and Y⁶, the number of nitrogen atoms is preferably two or more, more preferably three or more, and still more preferably four.

Among Y³, Y⁴, Y⁵ and Y⁶, Y³ and Y⁴ are preferably nitrogen atoms.

Examples of the aryl group having 6 to 18 carbon atoms represented by Ar¹, Ar², Ar³, Ar⁴ and Ar⁵ include a phenyl group, a biphenylyl group, a terphenylyl group, a naphthyl group, a phenanthryl group, an anthryl group, a fluorenyl group, a dimethylfluorenyl group, a spirofluorenyl group, a pyrenyl group, a fluoranthenyl group, a triphenylenyl group, a tetracenyl group, and a chrysenyl group. Among these, a phenyl group, a biphenylyl group and a naphthyl group are preferable. Among the substituents of the aryl group having 6 to 18 carbon atoms, examples of the fluoroalkyl group include a trifluoromethyl group, a pentafluoroethyl group, a heptafluoropropyl group, a nonafluorobutyl group, a undecafluoropentyl group, and a tridecafluorohexyl group. Among these, a trifluoromethyl group is preferable.

Examples of the heteroaryl group having 3 to 17 carbon atoms represented by Ar¹, Ar², and Ar³ include a pyridyl group, a bipyridyl group, a terpyridyl group, a phenylpyridyl group, a diphenylpyridyl group, a pyridylphenyl group, a pyrazyl group, a phenylpyrazyl group, a pyrazylphenyl group, a pyrimidyl group, a phenylpyrimidyl group, a diphenylpyrimidyl group, a pyrimidylphenyl group, a triazyl group, a phenyltriazyl group, a diphenyltriazyl group, a diphenyltriazylphenyl group, a quinolyl group, a phenylquinolyl group, a quinolylphenyl group, an isoquinolyl group, a phenylisoquinolyl group, a quinolylphenyl group, an azaanthryl group, a diazaanthryl group, a triazaanthryl group, a tetraazaanthryl group, an azaphenanthryl group, a diazaphenanthryl group, a triazaphenanthryl group, a tetraazaphenanthryl group, an azapyrenyl group, a diazapyrenyl group, a triazapyrenyl group, a tetraazapyrenyl group, an azafluoranthenyl group, a diazafluoranthenyl group, a triazafluoranthenyl group, a tetraazafluoranthenyl group, an azatriphenylenyl group, a diazatriphenylenyl group, a triazatriphenylenyl group, a tetraazatriphenylenyl group, a pentaazatriphenylenyl group, and a hexaazatriphenylenyl group. Among these, a pyridyl group and a pyridylphenyl group are preferable. Among the substituents of the heteroaryl group having 3 to 17 carbon atoms, examples of the fluoroalkyl group include a trifluoromethyl group, a pentafluoroethyl group, a heptafluoropropyl group, a nonafluorobutyl group, an undecafluoropentyl group, and a tridecafluorohexyl group. Among these, a trifluoromethyl group is preferable.

Ar¹ is preferably a hydrogen atom, a fluoro group, a cyano group, a phenyl group or a pyridyl group. Ar² and Ar³ are preferably a cyano group, a trifluoromethyl group, a fluoro group, or a phenyl group. As X¹, a divalent group represented by the formula (a) is preferable. In the formula (a), as Ar⁴ and Ar⁵, a cyano group, a trifluoromethyl group, a pyridyl group and a phenyl group are preferable.

Specific examples of the compound represented by the formula (1) or (2) include the following compounds (A-1) to (A-90).

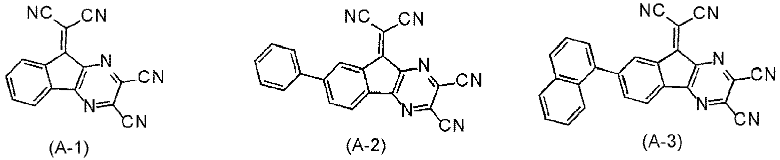

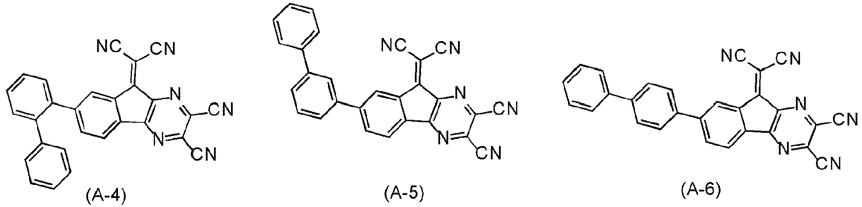

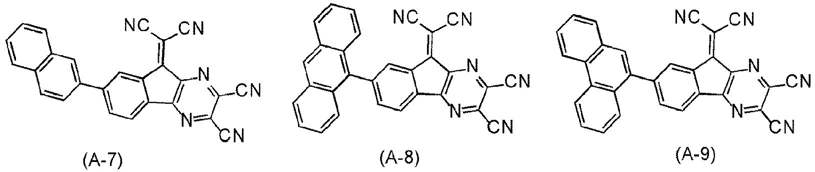

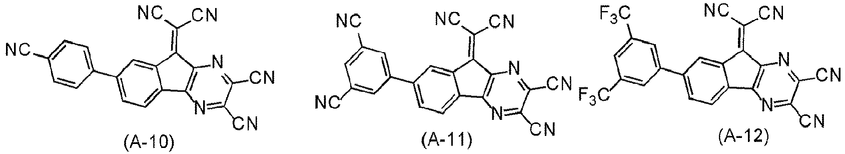

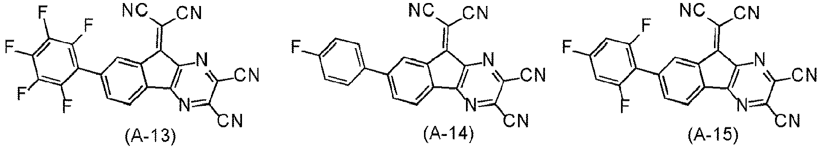

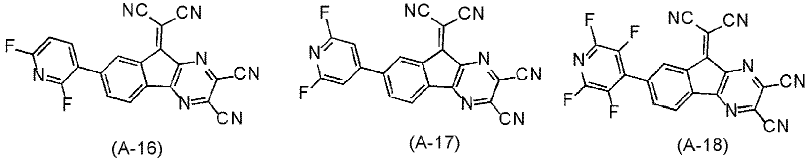

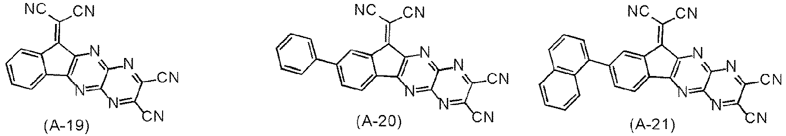

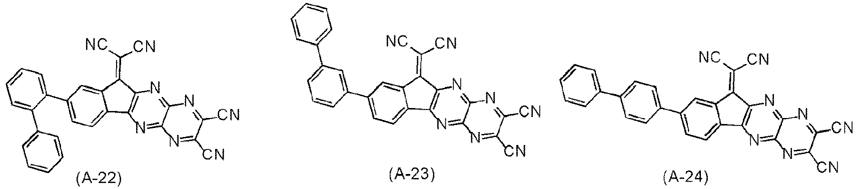

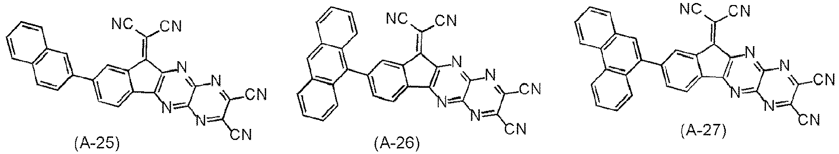

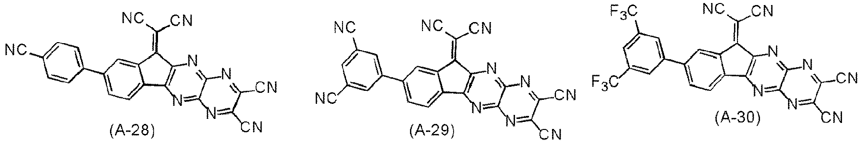

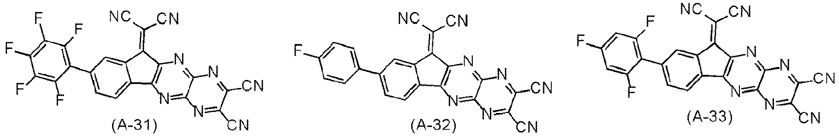

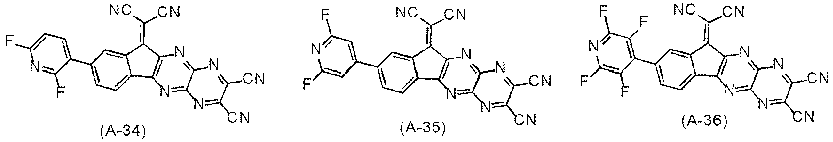

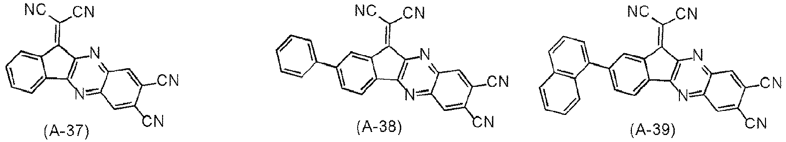

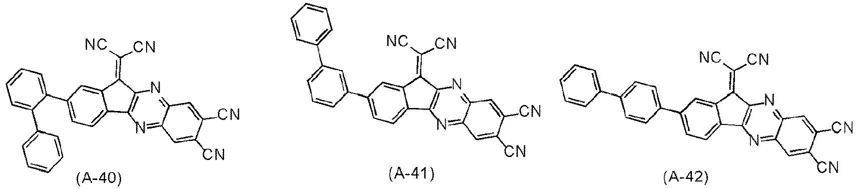

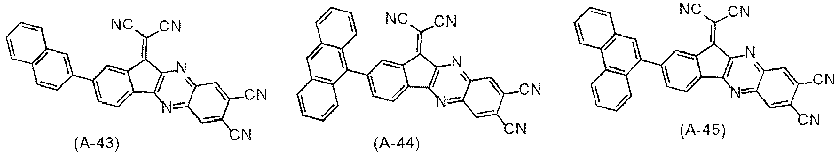

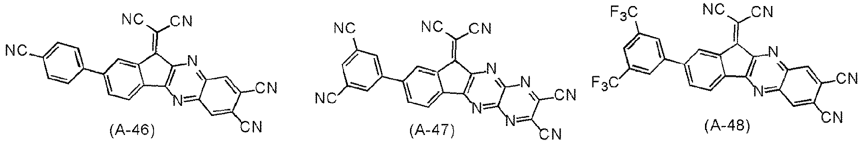

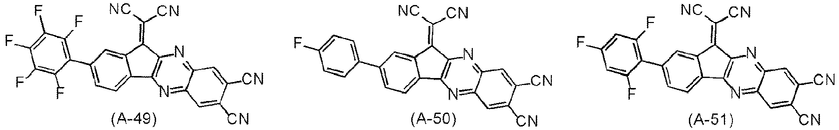

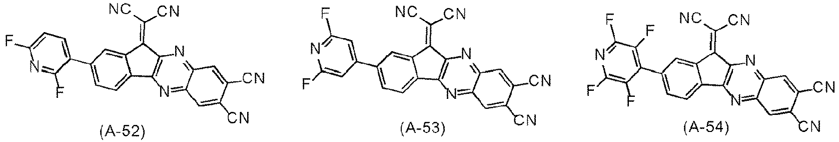

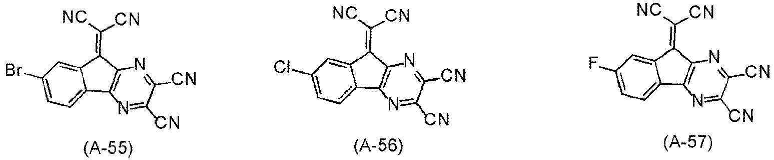

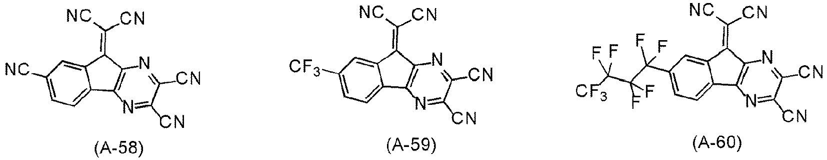

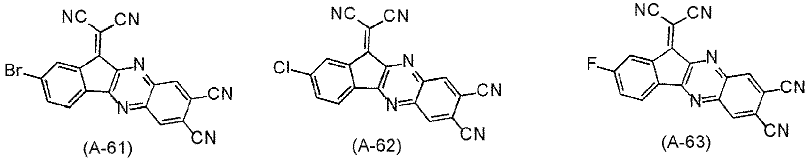

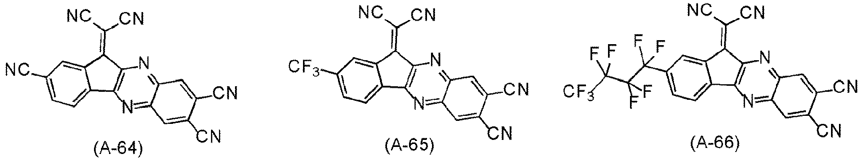

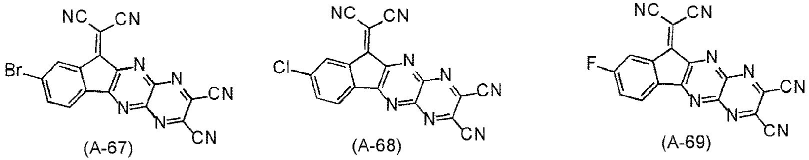

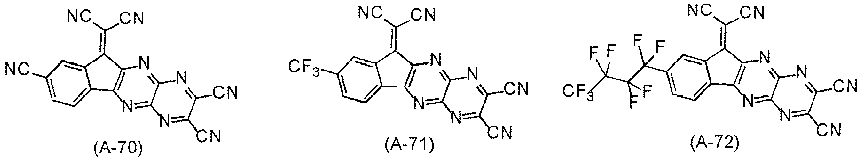

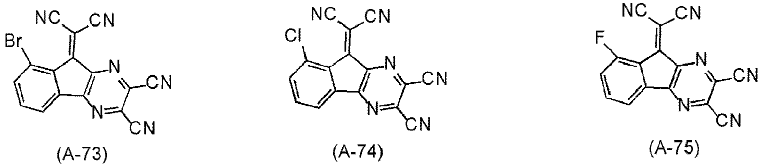

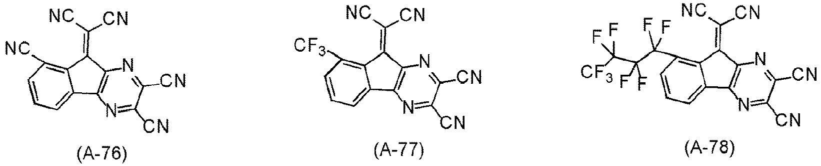

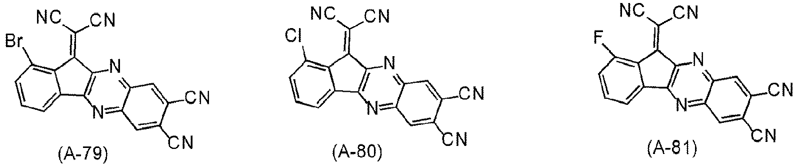

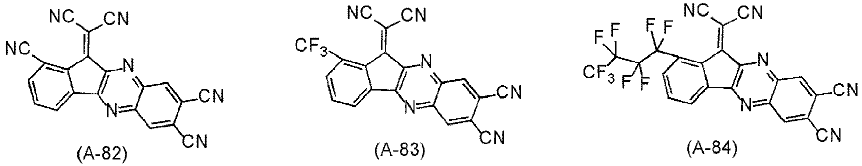

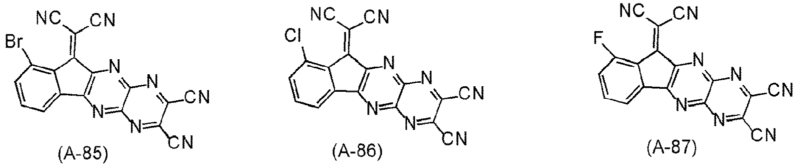

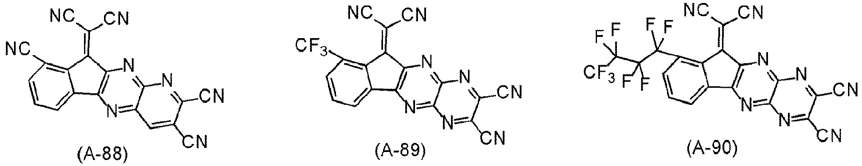

The light receiving element material containing the compound represented by the general formula (1) or (2) can be synthesized by a well-known method. For example, it can be synthesized by the method disclosed in WO 2008/072586 and the like.

### [Application of Light Receiving Element Material]

The light receiving element material of the present embodiment is preferably used as a light receiving element material for organic imaging element.

The light receiving element material of the present embodiment is used as a hole transport promoting material. The light receiving element material of the present embodiment is preferably used as a hole transport promoting material for light receiving element for organic imaging element. The hole transporting promoter material is a material constituting the hole transport promoting layer provided between the hole transporting layer responsible for transporting holes and the electrode to which the holes are transported. By providing the hole transport promoting layer, hole transport capability from the hole transporting layer to the electrode can be significantly improved.

### <Hole Transport Promoting Material>

The hole transport promoting material of the present embodiment includes a compound represented by the following general formula (1) or (2): 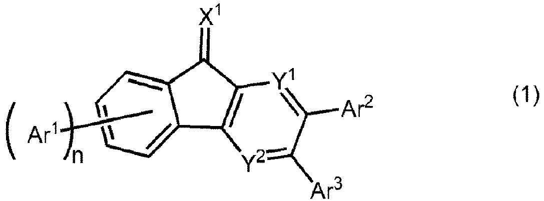 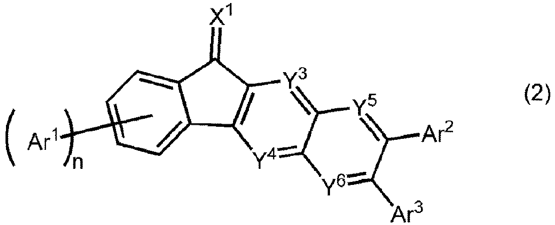 wherein
Y¹, Y², Y³, Y⁴, Y⁵ and Y⁶ each independently represent a nitrogen atom or C-H;
at least one of Y¹ or Y² is a nitrogen atom;
at least one of Y³, Y⁴, Y⁵ or Y⁶ is a nitrogen atom;
Ar¹, Ar² and Ar³ each independently represent
a hydrogen atom,
a cyano group,
a fluoro group,
a fluoroalkyl group,
an aryl group having 6 to 18 carbon atoms and being optionally substituted with a fluoro group, a fluoroalkyl group, or a cyano group; or
a heteroaryl group having 3 to 17 carbon atoms and being optionally substituted with a fluoro group, a fluoroalkyl group, or a cyano group;
n represents an integer of 1 to 4; and
X¹ is a divalent group represented by the following formula (a) or (b): 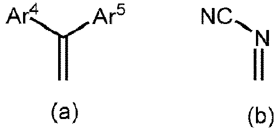 wherein
   Ar⁴ and Ar⁵ each independently represent
   a cyano group,
   a fluoro group,
   a fluoroalkyl group,
   an aryl group having 6 to 18 carbon atoms and being optionally substituted with a fluoro group, a fluoroalkyl group, or a cyano group; or
   a heteroaryl group having 3 to 17 carbon atoms and being optionally substituted with a fluoro group, a fluoroalkyl group, or a cyano group.

By providing the hole transport promoting layer including the hole transport promoting material represented by the general formula (1) or (2) between the hole transporting layer responsible for transporting holes and the electrode to which the holes are transported, the hole transport capability of the light receiving element can be significantly improved.

Preferred embodiments of the compound represented by the general formula (1) and the compound represented by the general formula (2) are the same as those of the compound represented by the general formula (1) and the compound represented by the general formula (2) used in the above-mentioned light receiving element material, and thus, the description thereof is omitted here.

### <Cyano Compounds>

The cyano compound of the present embodiment is represented by the following general formula (3), general formula (4), general formula (5) or general formula (6): 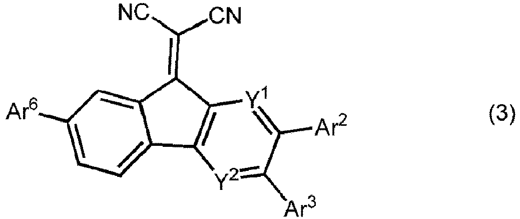 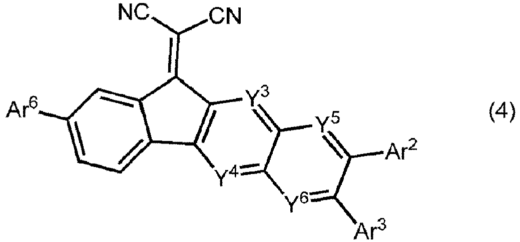 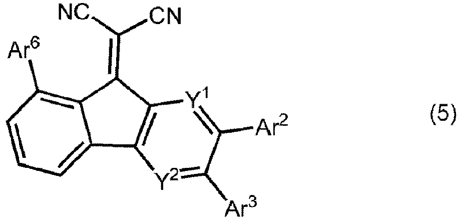 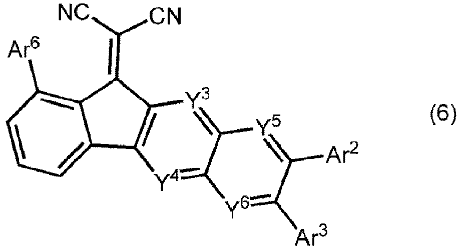 wherein
Y¹, Y², Y³, Y⁴, Y⁵ and Y⁶ each independently represent a nitrogen atom or C-H;
at least one of Y¹ or Y² is a nitrogen atom;
at least one of Y³, Y⁴, Y⁵ or Y⁶ is a nitrogen atom; and
Ar⁶ represents:
   a cyano group,
   a fluoro group, a chloro group, a bromo group, an iodine group a fluoroalkyl group,
   an aryl group having 6 to 18 carbon atoms substituted with a fluoro group, a fluoroalkyl group, or a cyano group; or a heteroaryl group having 3 to 17 carbon atoms and being optionally substituted with a fluoro group, a fluoroalkyl group, or a cyano group;
   provided that when Y⁵ and Y⁶ are C-H, Ar⁶ may be a hydrogen atom; and
   Ar² and Ar³ each independently represent:
      a hydrogen atom,
      a cyano group,
      a fluoro group,
      a fluoroalkyl group,
      an aryl group having 6 to 18 carbon atoms and being optionally substituted with a fluoro group, a fluoroalkyl group, or a cyano group; or
      a heteroaryl group having 3 to 17 carbon atoms and being optionally substituted with a fluoro group, a fluoroalkyl group, or a cyano group.

By providing the layer containing the cyano compound represented by the general formula (3), the general formula (4), the general formula (5), or the general formula (6) between the hole transporting layer responsible for transporting holes and the electrode to which the holes are transported, the hole transport capability of the light receiving element can be significantly improved.

Preferred embodiments of the cyano compound represented by the general formula (3), the general formula (4), the general formula (5), or the general formula (6) are as follows because the hole transport capability of the light receiving element can be significantly improved.

Y¹ and Y² are each preferably a nitrogen atom.

Among Y³, Y⁴, Y⁵ and Y⁶, the number of nitrogen atoms is preferably two or more, more preferably three or more, and still more preferably four.

Among Y³, Y⁴, Y⁵ and Y⁶, Y³ and Y⁴ are preferably nitrogen atoms.

Examples of the aryl group having 6 to 18 carbon atoms represented by Ar², Ar³, and Ar⁶ include a phenyl group, a biphenylyl group, a terphenylyl group, a naphthyl group, a phenanthryl group, an anthryl group, a fluorenyl group, a dimethylfluorenyl group, a spirofluorenyl group, a pyrenyl group, a fluoranthenyl group, a triphenylenyl group, a tetracenyl group, and a chrysenyl group. Among these, a phenyl group, a biphenylyl group and a naphthyl group are preferable. Among substituents of the aryl group having 6 to 18 carbon atoms, examples of the fluoroalkyl group include a trifluoromethyl group, a pentafluoroethyl group, a heptafluoropropyl group, a nonafluorobutyl group, a undecafluoropentyl group, and a tridecafluorohexyl group. Among these, a trifluoromethyl group is preferable.

Examples of the heteroaryl group having 3 to 17 carbon atoms represented by Ar¹, Ar², Ar³, Ar⁴ and Ar⁵ include a pyridyl group, a bipyridyl group, a terpyridyl group, a phenylpyridyl group, a diphenylpyridyl group, a pyridylphenyl group, a pyrazyl group, a phenylpyrazyl group, a pyrazylphenyl group, a pyrimidyl group, a phenylpyrimidyl group, a diphenylpyrimidyl group, a pyrimidylphenyl group, a triazyl group, a phenyltriazyl group, a diphenyltriazyl group, a diphenyltriazylphenyl group, a quinolyl group, a phenylquinolyl group, a quinolylphenyl group, an isoquinolyl group, a phenylisoquinolyl group, a quinolylphenyl group, an azaanthryl group, a diazaanthryl group, a triazaanthryl group, a tetraazaanthryl group, an azaphenanthryl group, a diazaphenanthryl group, a triazaphenanthryl group, a tetraazaphenanthryl group, an azapyrenyl group, a diazapyrenyl group, a triazapyrenyl group, a tetraazapyrenyl group, an azafluoranthenyl group, a diazafluoranthenyl group, a triazafluoranthenyl group, a tetraazafluoranthenyl group, an azatriphenylenyl group, a diazatriphenylenyl group, a triazatriphenylenyl group, a tetraazatriphenylenyl group, a pentaazatriphenylenyl group, and a hexaazatriphenylenyl group. Among these, a pyridyl group and a pyridylphenyl group are preferable. Among the substituents of the heteroaryl group having 3 to 17 carbon atoms, examples of the fluoroalkyl group include a trifluoromethyl group, a pentafluoroethyl group, a heptafluoropropyl group, a nonafluorobutyl group, a undecafluoropentyl group, and a tridecafluorohexyl group. Among these, a trifluoromethyl group is preferable.

Ar² and Ar³ are preferably a cyano group, a trifluoromethyl group, a fluoro group, or a phenyl group. Ar⁶ is preferably a fluoro group, a bromo group, a cyano group, a phenyl group or a pyridyl group.

### <Organic Receiving Element>

The organic light receiving element of the present embodiment includes a first electrode and a second electrode, and
has an organic layer between the first electrode and the second electrode,
the organic layer includes at least a light receiving layer, a hole transporting layer, and a hole transport promoting layer, and
the hole transport promoting layer contains a compound having a partial structure represented by the following formula (Q) or a compound represented by the following formula (1) or (2): 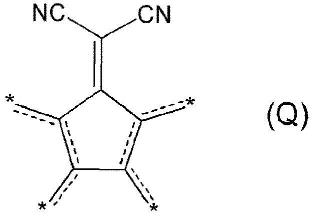 wherein portions in which solid lines and broken lines are parallel each independently represent a single bond or a double bond, and * represents a bond; 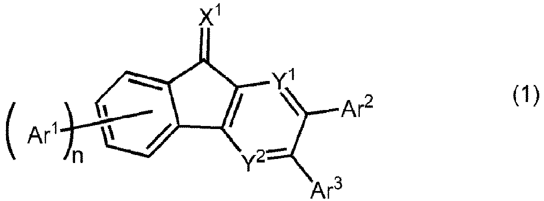 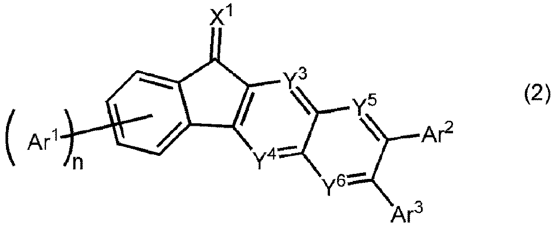 wherein
   Y¹, Y², Y³, Y⁴, Y⁵ and Y⁶ each independently represent a nitrogen atom or C-H;
   at least one of Y¹ or Y² is a nitrogen atom;
   at least one of Y³, Y⁴, Y⁵ or Y⁶ is a nitrogen atom;
   Ar¹, Ar² and Ar³ each independently represent:
      a hydrogen atom,
      a cyano group,
      a fluoro group,
      a fluoroalkyl group,
      an aryl group having 6 to 18 carbon atoms and being optionally substituted with a fluoro group, a fluoroalkyl group, or a cyano group; or
      a heteroaryl group having 3 to 17 carbon atoms and being optionally substituted with a fluoro group, a fluoroalkyl group, or a cyano group;
      n represents an integer of 1 to 4; and
      X¹ is a divalent group represented by the following formula (a) or (b): 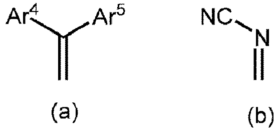 wherein
         Ar⁴ and Ar⁵ each independently represent:
         a cyano group,
         a fluoro group,
         a fluoroalkyl group,
         an aryl group having 6 to 18 carbon atoms and being optionally substituted with a fluoro group, a fluoroalkyl group, or a cyano group; or
         a heteroaryl group having 3 to 17 carbon atoms and being optionally substituted with a fluoro group, a fluoroalkyl group, or a cyano group.

By providing the hole transport promoting layer containing a compound having a partial structure represented by a general formula (Q) or a compound represented by the general formula (1) or (2) between the hole transporting layer, which is responsible for transporting holes, and the first electrode to which the holes are transported, the hole transport capability of the light receiving element can be significantly improved.

The compound having a partial structure represented by the general formula (Q) is preferably a compound having a condensed ring structure of three or more rings, and more preferably is bonded to a nitrogen atom or a carbon atom at the bond.

Preferred embodiments of the compound having a partial structure represented by the general formula (Q) are the same as the compounds (A-1) to (A-90) exemplified above as compounds used for the light receiving element material.

Preferred embodiments of the compound represented by the general formula (1) and the compound represented by the general formula (2) are the same as those of the compound represented by the general formula (1) and the compound represented by the general formula which are used in the above-mentioned light receiving element material, and the description thereof is omitted here.

A laminate structure of the organic light receiving element of the present embodiment is not particularly limited, but examples thereof include the structures (i) and (ii):
(i): first electrode/hole transport promoting layer/hole transporting layer/light receiving layer/second electrode; and
(ii): first electrode/hole transport promoting layer/hole transporting layer/light receiving layer/electron transporting layer/second electrode.

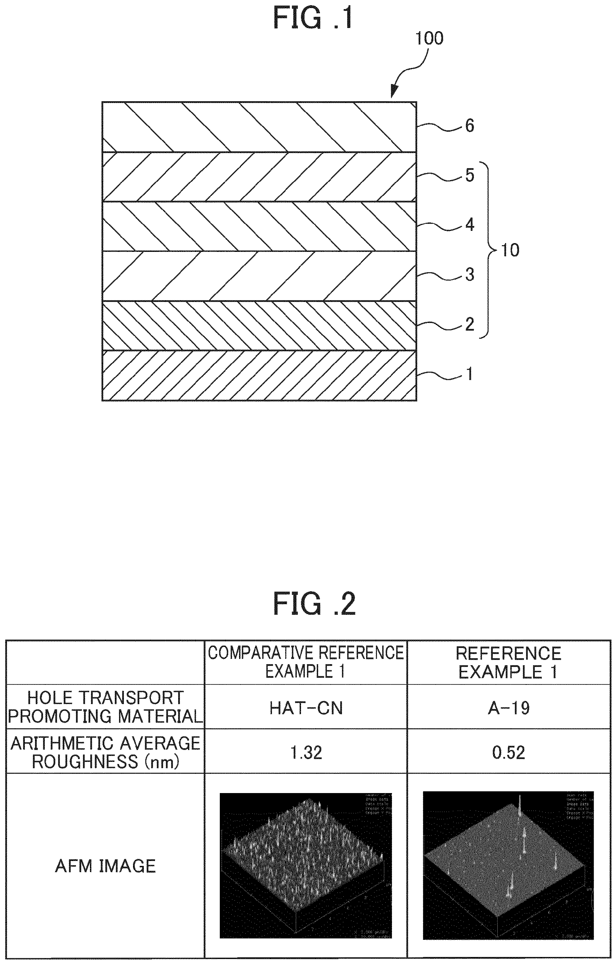

Hereinafter, the organic light receiving element of the present embodiment will be described in detail with reference to FIG. 1 by taking the configuration (ii) as an example. FIG. 1 is a schematic cross-sectional view showing an example of a laminated structure of an organic light receiving element of the present embodiment.

An organic light receiving element 100 includes a first electrode 1, a hole transport promoting layer 2, a hole transporting layer 3, a light receiving layer 4, an electron transporting layer 5, and a second electrode 6 in this order. However, some of these layers may be omitted, and conversely, other layers may be added. Among the above layers, the hole transport promoting layer 2, the hole transporting layer 3, the light receiving layer 4, and the electron transporting layer 5 constitute an organic layer 10.

In the organic light receiving element 100 shown in FIG. 1, light is incident from below the transparent first electrode 1 and is received by the light receiving layer 4. In addition, a voltage is applied to the organic light receiving element 100 so that, out of charges (holes and electrons) generated by photoelectric conversion in the light receiving layer 4, holes are transferred to the first electrode 1 and electrons are transferred to the second electrode 6. That is, the first electrode 1 is a hole trapping electrode, and the second electrode 6 is an electron trapping electrode. In FIG. 1, a substrate provided on the lower surface of the first electrode 1 is omitted. The substrate is not particularly limited, and examples thereof include a glass plate, a quartz plate, and a plastic plate. In the case of a structure in which light is incident from the substrate side, the substrate is transparent to wavelengths of light. The above-described layers will be described below.

### [First Electrode 1]

The first electrode 1 is provided on a substrate.

In the case of an organic light receiving element in which light passes through the first electrode 1 and is incident on the light receiving layer 4, the first electrode is formed of a transparent material which transmits or substantially transmits the light.

The transparent material used for the first electrode 1 is not particularly limited, and examples thereof include indium-tin oxide (ITO), indium-zinc oxide (IZO), tin oxide, aluminum doped tin oxide, magnesium-indium oxide, nickel-tungsten oxide, other metal oxides, metal nitrides such as gallium nitride, metal selenides such as zinc selenide, etc. and metal sulfides such as zinc sulfide.

In the case of the organic light receiving element having a configuration in which light is incident on the light receiving layer 4 only from the second electrode 6 side, the transmission characteristic of the first electrode 1 is not important. Accordingly, examples of the material used for the first electrode in this case include gold, iridium, molybdenum, palladium, and platinum.

### [Hole Transport Promoting Layer 2]

The hole transport promoting layer 2 is provided between the first electrode 1 and a hole transporting layer 3 described below. The hole transport promoting layer 2 is provided to promote hole transport from the hole transporting layer 3 to the first electrode 1. The hole transport promoting layer 2 contains a compound having a partial structure represented by the above general formula (Q) or a compound represented by the general formula (1) or (2). Compounds other than the above compounds may also be contained. Examples of the compound that can be contained in the hole transport promoting layer 2 include conventionally known hole transporting materials, and examples thereof include compounds exemplified in the hole transporting layer 3 described below.

### [Hole Transporting Layer 3]

The hole transporting layer 3 is provided between the hole transport promoting layer 2 and the light receiving layer 4.

The hole transporting layer 3 has a role of transporting holes generated in the light receiving layer 4 from the light receiving layer 4 to the first electrode 1, and a role of blocking electrons generated in the light receiving layer 4 from moving to the first electrode 1 side. Further, depending upon the application, the hole transporting layer 3 may have a role of blocking electron injection from the first electrode 1.

The hole transporting layer 3 may have a single layer structure made of one type or two or more types of materials, or may have a laminated structure made of a plurality of layers of the same composition or different compositions.

The hole transporting layer 3 preferably contains a known hole transporting material. Known hole transporting materials include: aromatic tertiary amine compounds, naphthalene compounds, anthracene compounds, tetracene compounds, pentacene compounds, phenanthrene compounds, pyrene compounds, perylene compounds, fluorene compounds, carbazole compounds, indole compounds, pyrrole compounds, picene compounds, thiophene compounds, benzotrifuran compounds, benzotrithiophene compounds, naphthodithiophene compounds, naphthothienothiophene compounds, benzodifuran compounds, benzodithiophene compounds, benzothiophene compounds, naphthobisbenzothiophene compounds, chrysenodithiophene compounds, benzothienobenzothiophene compounds, and indolocarbazole compounds.

Among these, fluorene compounds, naphthodithiophene compounds, naphthienothiophene compounds, benzodifuran compounds, benzothiophene compounds, naphthobisbenzothiophene compounds, chrysenodithiophene compounds, benzothienobenzothiophene compounds, indolocarbazole compounds, and the like are preferable, and fluorene compounds, chrysenodithiophene compounds, benzothienobenzothiophene compounds, and an indocarbazole compounds are particularly preferable.

Specific examples of known hole transporting materials include: N,N'-bis(1-naphthyl)-1,1-biphenyl-4,4'-diamine (NPD), 9,9'-(9,9'-spirobi[9H-fluorene]-2,7'-diyl)bis[9H-carbazole], 2,7-diphenyl[1]benzothieno[3,2-b][1]benzothiophene (DiPh-BTBT), benzo[1,2-b:3,4-b':5,6-b"]trifuran compounds, benzo[1,2-b:3,4-b':5,6-b"]trithiophene compounds, naphtho[1,2-b:5,6-b']dithiophene, naphtho[2,3-b]naphtho[2',3':4,5]thieno[2,3-d]thiophene, benzo[1,2-b:4,5-b']difuran, benzo[1,2-b:4,5-b']dithiophene, benzo[1,2-b:4,5-b']bis[1]benzothiophene, naphtho[1,2-b:5,6-b']bis[1]benzothiophene, chryseno[1,2-b:8,7-b']dithiophene, [1]benzothieno[3,2-b][1]benzothiophene, and compounds shown below: (ic-1), (ic-2) and (ic-3).

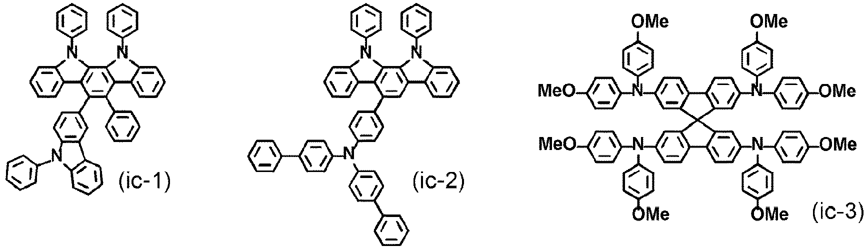

### [Light Receiving Layer 4]

The light receiving layer 4 is provided between the hole transporting layer 3 and an electron transporting layer 5 described below.

Examples of the material for the light receiving layer 4 include a material having a photoelectric conversion function.

The light receiving layer 4 may have a single layer structure made of one type or two or more types of materials, or may have a laminated structure made of a plurality of layers of the same composition or different compositions.

Examples of the material to be used for the light receiving layer 4 having a single layer structure made of one type of material include (1) coumarin and its derivatives, quinacridone and its derivatives, and phthalocyanine and its derivatives.

Examples of the material to be used for the light receiving layer 4 having a single layer structure made of two types of materials include a combination of (i) coumarin and its derivatives, quinacridone and its derivatives, and phthalocyanine and its derivatives, and (ii) fullerene and its derivatives. The light receiving layer 4 made of these materials may be formed by mixing powders in advance, and then depositing the powders in a mixed state, or may be formed by co-deposition at any ratio.

Examples of the material used for the light receiving layer 4, which is a single layer structure made of three types of materials, include a combination of (i) coumarin and its derivatives, quinacridone and its derivatives, and phthalocyanine and its derivatives, (ii) fullerene and its derivatives, and (iii) a hole transporting material. The light receiving layer 4 made of these materials may be formed by mixing powders in advance, and then depositing the powders in a mixed state, or may be formed by co-deposition at any ratio.

Specific examples of the coumarin derivatives (i) include coumarin 6 and coumarin 30. Specific examples of the quinacridone derivatives include N,N-dimethylquinacridone. Specific examples of the phthalocyanine derivatives include boron subphthalocyanine chloride and boron subnaphthalocyanine chloride (SubNC).

Specific examples of the fullerene and its derivatives (ii) include [60] fullerene, [70] fullerene, and [6,6]-phenyl-C61-methyl butyrate ([60] PCBM).

Preferred compounds and specific examples of the hole transporting material (iii) include the same compounds as those described in the hole transporting layer 3.

Further, the material having a photoelectric conversion function is not necessarily contained in the light receiving layer alone. For example, the material having the photoelectric conversion function may be contained in a layer (hole transporting layer 3 or electron transporting layer 5) adjacent to the light receiving layer 4.

### [Electron Transporting Layer 5]

The electron transporting layer 5 is provided between the light receiving layer 4 and the second electrode 6 described below.

The electron transporting layer 5 has a role of transporting electrons generated in the light receiving layer 4 to the second electrode 6 and a role of blocking the holes from moving from the second electrode 6, i.e., an electron transport destination, to the light receiving layer 4. Depending on the application, the electron transporting layer 5 may have a role of blocking hole injection from the second electrode 6.

The electron transporting layer 5 may contain a known electron transporting material. Examples of known electron transporting materials include: fullerene, fullerene derivatives, bis(8-hydroxyquinolinato)manganese, tris(8-hydroxyquinolinato)aluminum, tris(2-methyl-8-hydroxyquinolinato)aluminum, BCP(2,9-dimethyl-4,7-diphenyl-1,10-phenanthroline), Bphen(4,7-diphenyl-1,10-phenanthroline), BAlq(bis(2-methyl-8-quinolinolato)-4-(phenylphenolato)aluminum), 4,6-bis(3,5-di(pyridin-4-yl)phenyl)-2-methylpyrimidine, N,N'-diphenyl-1,4,5,8-naphthalene tetracarboxylic diimide, and N,N'-di(4-pyridyl)-1,4,5,8-naphthalene tetracarboxylic diimide.

The electron transporting layer 5 may have a single layer structure made of one type or two or more types of materials, or may have a laminated structure made of a plurality of layers of the same composition or different compositions.

### [Second Electrode 6]

The second electrode 6 is provided on the electron transporting layer 5.

Examples of the material for the second electrode 6 include: indium-tin oxide (ITO), indium-zinc oxide (IZO), sodium, a sodium-potassium alloy, magnesium, lithium, a magnesium/copper mixture, a magnesium/silver mixture, a magnesium/aluminum mixture, a magnesium/indium mixture, an aluminum/aluminum oxide (Al₂O₃) mixture, indium, a lithium/aluminum mixture, gold, platinum, a rare earth metal, molybdenum oxide, and the like. Note that the first electrode 1 and the second electrode 6 may be the same or different.

### [Method of forming Each Layer]

Each layer except for the first electrode 1 and the second electrode 6 described above can be produced by forming a thin film from materials for the layer (together with a material such as a binder resin and a solvent, if necessary) by a well-known method such as a vacuum vapor deposition method, a spin coating method, a casting method, or an LB method (Langmuir-Blodgett method).

The film thickness of each layer formed in this manner is not particularly limited and can be appropriately selected depending on the circumstances, and is usually in the range of 5 nm or more and 5 µm or less.

The first electrode 1 and the second electrode 6 can be produced by forming a thin film from electrode materials by a method such as vapor deposition or sputtering. A pattern may be formed through a mask having a desired shape during vapor deposition or sputtering, or a pattern having a desired shape may be formed by photolithography after a thin film is formed by vapor deposition or sputtering.

The thicknesses of the first electrode 1 and the second electrode 6 are preferably 1 um or less, and more preferably 10 nm or more and 200 nm or less.

The materials constituting the first electrode 1 and the second electrode 6 may be exchanged between them (also referred to as a reverse structure) as necessary. In the case of such a structure, light passes through the second electrode 6 and is incident on the light receiving layer 4 in the obtained organic light receiving element.

The imaging element including the organic light receiving element of the present embodiment can be applied to, for example, an imaging element for a digital camera or a digital video camera, and an imaging element built in a mobile phone or the like.

### EXAMPLES

Hereinafter, the present invention will be described in more detail based on Examples, but the present invention should not be construed as being limited to these Examples.

### <Synthesis Examples>

### [Synthesis Example 1: Synthesis of Compound (A-37)]

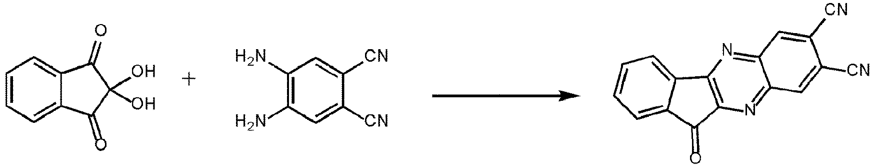

Ninhydrin (2.00 g, 11.2 mmol) and 1,2-diamino-4,5-dicyanopyrazine (1.78 g, 11.2 mmol) were suspended in a mixed solvent of 58 mL of water, 88 mL of ethanol and 4 mL of acetic acid under an argon stream, and heated at 80°C for 17 hours. After cooling to room temperature, water was added to the reaction mixture. The resulting solid was collected by filtration and purified by recrystallization with toluene to obtain 11-oxo-11H-indeno[1,2-b]quinoxaline-7,8-dicarbonitrile represented by the above formula (2.61 g, yield 82%).

¹HNMR (DMSO-d6) δ (ppm): 7.84 (t, J=7.5Hz, 1H), 7.96 (dd, J=7.6, 1.2 Hz, 1H), 8.00 (brd, J=7.6 Hz, 1H), 8.19 (brd, J=7.5Hz, 1H), 8.99 (s, 1H), 9.09 (s, 1H)

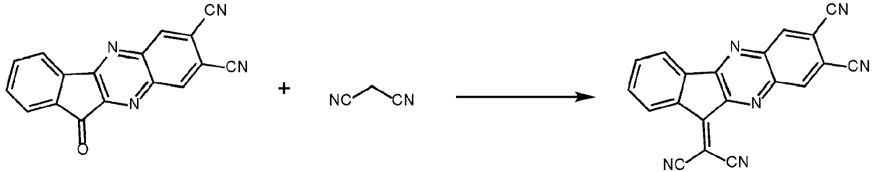

Under an argon stream, 11-oxo-11H-indeno[1,2-b]quinoxaline-7,8-dicarbonitrile (2.00 g, 7.09 mmol) obtained above and malononitrile (0.56 g, 8.51 mmol) were suspended in 142 mL of DMF and stirred at room temperature for 17 hours. To the resulting reaction mixture, 100 mL of water was added. The resulting solid was collected by filtration and purified by recrystallization with a mixed solvent of toluene and methanol to obtain a desired compound (A-37) (2.20 g, yield 94%).
¹HNMR (DMSO-d6) δ (ppm): 7.96 (brs,2H), 8.25 (brd, J=7.1 Hz, 1H), 8.52 (brd, J=7.0 Hz,1H), 8.97 (brs, 1H), 9.03 (brs, 1H)

### [Synthesis Example 2: Synthesis of Compound (A-55)]

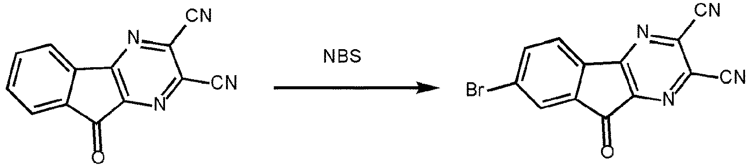

Under an argon stream, 9-oxo-9H-indeno[1,2-b]pyrazine-1,3-dicarbonitrile (10.0 g, 43.1 mmol) was suspended in a mixed solvent of 120 mL trifluoroacetic acid and 50 mL sulfuric acid and cooled to 0°C. NBS (8.43 g, 47.4 mmol) was added to the resulting mixed solution and stirred for 20 minutes, followed by stirring at room temperature for 22 hours. To the resulting mixed solution, 300 mL of water was added, and the resulting solid was collected by filtration. The obtained solid was washed with acetonitrile to obtain 7-bromo-9-oxo-9H-indeno[1,2-b]pyrazine-1,3-dicarbonitrile represented by the above formula (3.18 g, yield 24%).
¹HNMR(CDCl₃) δ (ppm): 7.94 (d, J=8.1 Hz, 1H), 8.00 (dd, J=8.0, 1.8 Hz,1H), 8.11 (dd, J=1.8, 0.5 Hz, 1H)

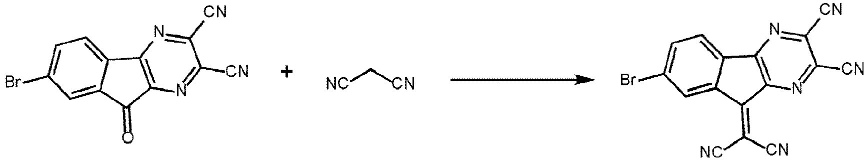

Under an argon stream, 7-bromo-9-oxo-9H-indeno[1,2-b]pyrazine-1,3-dicarbonitrile (0.60 g, 1.93 mmol) obtained above and malononitrile (0.15 g, 2.31 mmol) were suspended in 100 mL of DMF and stirred at room temperature for 17 hours. To the resulting reaction mixture, 300 mL of water was added. The resulting solids were collected by filtration and purified by recrystallization with a mixed solvent of toluene and methanol to obtain a desired compound (A-55) (0.45 g, yield 65%).
¹HNMR (CDCl₃) δ (ppm): 8.01 (s, 1H), 8.01 (s, 1H), 8.74 (s, 1H)

### <Production and Evaluation 1 of Hole-Only Device>

### [Element Example 1]

A hole-only device having a structure consisting of first electrode/hole injection layer/hole transporting layer/hole transport promoting layer/second electrode was produced, and the hole transport characteristics of the device were evaluated.

### (First Electrode)

As a substrate provided with the first electrode on the surface thereof, a glass substrate with an ITO transparent electrode in which an ITO film (film thickness: 110 nm) had been patterned in a stripe shape was prepared, and this substrate was washed with isopropyl alcohol, followed by surface treatment by ozone ultraviolet cleaning.

### (Preparation of Vacuum Vapor Deposition)

Among two surfaces of the substrate subjected to the surface treatment, on the surface where the ITO film was formed, each layer was vacuum-deposited by a vacuum vapor deposition method to form a laminated layer.

First, the glass substrate was introduced into a vacuum vapor deposition tank and pressure inside the tank was reduced to 1.0×10⁻⁴ Pa. Then, each layer was formed in the following order in accordance with the film forming conditions of the respective layers.

### (Production of Hole Injection Layer)

MoOs was deposited on the ITO film to a film thickness of 1 nm to produce a hole injection layer.

### (Production of Hole transporting layer)

Sublimation purified N,N'-bis(1-naphthyl)-1,1-biphenyl-4,4'-diamine (NPD) shown below was deposited to a film thickness of 30 nm to produce a hole transporting layer.

### (Production of Hole Transport Promoting Layer)

A sublimation purified compound (A-19) shown below was deposited to a film thickness of 15 nm to produce a hole transport promoting layer. Note that the compound (A-19) was synthesized according to the method disclosed in WO 2008/072586.

### (Production of Second Electrode)

Ag was deposited to a film thickness of 80 nm to produce a second electrode having a two-layer structure.

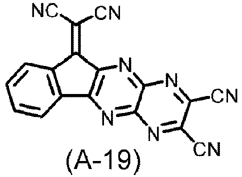

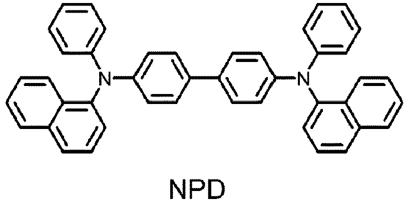

### (Evaluation of Hole Transport Capability of Hole-Only Device)

Positive and negative electric fields were applied to the first electrode and the second electrode of the hole-only device of Element Example 1, respectively, and a voltage value at a current density of 10 mA/cm² was measured. The obtained results are shown in Table 1.

### [Element Example 2]

A hole-only device was produced in the same manner as in Element Example 1 except that a compound (A-1) shown below was used instead of the compound (A-19) used in production of the hole transport promoting layer Element Example 1. Note that the compound (A-1) was synthesized according to the method disclosed in WO 2008/072586. The hole transport capability of the obtained hole-only device was evaluated in the same manner as in Example 1. The results are shown in Table 1.

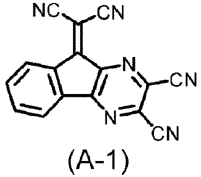

### [Element Comparative Example 1]

A hole-only device was produced in the same manner as in Example 1, except that the following triazine derivative (NPT) was used instead of the compound (A-19) used in the production of the hole transport promoting layer in Example 1. The hole transport capability of the obtained hole-only device was evaluated in the same manner as in Example 1. The results are shown in Table 1.

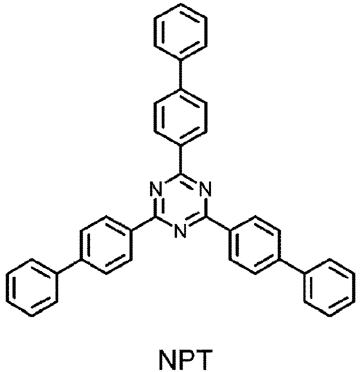

### [Element Comparative Example 2]

A hole-only device was produced in the same manner as in Element Example 1 except that the device did not have a hole transport promoting layer. The hole transport capability of the obtained hole-only device was evaluated in the same manner as in Element Example 1. The results are shown in Table 1.

### [Element Comparative Example 3]

A hole-only device was produced in the same manner as in Element Example 1, except that 2,3,6,7,10,11-hexacyano-1,4,5,8,9,12-hexaazatriphenylene (HAT-CN) shown below was used instead of the compound (A-19) used in the production of the hole transport promoting layer in Element Example 1. The hole transport capability of the obtained hole-only device was evaluated in the same manner as in Element Example 1. The results are shown in Table 1.

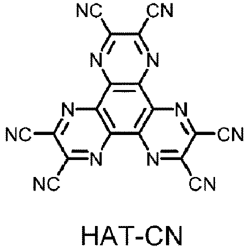

**[Table 1]**

| | Hole transporting material | Hole transport promoting material | Voltage (V) |
|---|---|---|---|
| Comparative Example 1 | NPD | None | 3.05 |
| Comparative Example 2 | NPD | NPT | 3.58 |
| Comparative Example 3 | NPD | HAT-CN | 3.71 |
| Example 1 | NPD | A-19 | 1.17 |
| Example 2 | NPD | A-1 | 1.45 |

### <Production and Evaluation 2 of Hole-Only Device>

### [Element Example 3]

A hole-only device having a structure consisting of first electrode/hole injection layer/hole transporting layer/hole transport promoting layer/second electrode was produced, and the hole transport characteristics of the device were evaluated.

### (First Electrode)

As a substrate provided with the first electrode on the surface thereof, a glass substrate with an ITO transparent electrode in which an ITO film (film thickness: 110 nm) had been patterned in a stripe shape was prepared, and this substrate was washed with isopropyl alcohol, followed by surface treatment by ozone ultraviolet cleaning.

### (Preparation of Vacuum Vapor Deposition)

Among two surfaces of the substrate subjected to the surface treatment, on the surface where the ITO film was formed, each layer was vacuum-deposited by a vacuum vapor deposition method to form a laminated layer.

First, the glass substrate was introduced into a vacuum vapor deposition tank and pressure inside the tank was reduced to 1.0×10⁻⁴ Pa. Then, each layer was formed in the following order in accordance with the respective film forming conditions.

### (Production of Hole Injection Layer)

MoOs was deposited on the ITO film to a film thickness of 1 nm to produce a hole injection layer.

### (Production of Hole Transporting Layer)

The above-mentioned sublimation purified N,N'-bis(1-naphthyl)-1,1-biphenyl-4,4'-diamine (NPD) was deposited to a film thickness of 100 nm to produce a hole transporting layer.

### (Production of Hole Transport Promoting Layer)

The above-mentioned sublimation purified compound (A-19) was deposited to a film thickness of 10 nm to produce a hole transport promoting layer.

### (Production of Second Electrode)

Au was deposited to a film thickness of 80 nm to produce a second electrode having a two-layer structure.

### (Evaluation of transport Capability of Hole-Only Device)

Positive and negative electric fields were applied to the first electrode and the second electrode of the hole-only device of Element Example 3, respectively, and a voltage value at a current density of 10 mA/cm² was measured. The obtained results are shown in Table 2.

### [Element Example 4]

A hole-only device was produced in the same manner as in Element Example 3 except that the above-described compound (A-1) was used instead of the compound (A-19) used in the production of the hole transport promoting layer in Element Example 1. The hole transport capability of the obtained hole-only device was evaluated in the same manner as in Example 3. The results are shown in Table 2.

### [Element Comparative Example 4]

A hole-only device was produced in the same manner as in Element Example 3, except that the aforementioned NPT was used instead of the compound (A-19) used in the production of the hole transport promoting layer in Element Example 3. The hole transport capability of the obtained hole-only device was evaluated in the same manner as in Element Example 3. The results are shown in Table 2.

### [Element Comparative Example 5]

A hole-only device was produced in the same manner as in Element Example 3 except that the device did not have a hole transport promoting layer. The hole transport capability of the obtained hole-only device was evaluated in the same manner as in Element Example 3. The results are shown in Table 2.

### [Element Comparative Example 6]

A hole-only device was produced in the same manner as in Element Example 3 except that the above-described HAT-CN was used instead of the compound (A-19) used in the production of the hole transport promoting layer in Example 3. The hole transport capability of the obtained hole-only device was evaluated in the same manner as in Element Example 3. The results are shown in Table 2.

**[Table 2]**

| | Hole transporting material | Hole transport promoting material | Voltage (V) |
|---|---|---|---|
| Comparative Example 4 | NPD | None | 3.01 |
| Comparative Example 5 | NPD | NPT | 2.60 |
| Comparative Example 6 | NPD | HAT-CN | 1.23 |
| Example 3 | NPD | A-19 | 1.06 |
| Example 4 | NPD | A-1 | 1.19 |

From the results of Tables 1 and 2, it can be understood that the hole transport capability significantly improved by using A-1 or A-19 as the hole transport promoting material in combination with the hole transporting material.

### <Production and Evaluation of Photoelectric Conversion Element>

### [Element Example 5]

A photoelectric conversion element 100 having a laminated structure consisting of substrate/second electrode 6/electron transporting layer 5/photoelectric conversion layer 4/hole transporting layer 3/hole transport promoting layer 2/first electrode 1 was produced, and dark current and external quantum efficiency of the photoelectric conversion element were evaluated.

### (Preparation of Substrate and Second Electrode 6)

As a substrate provided with a second electrode on the surface thereof, a glass substrate with an ITO transparent electrode in which an indium-tin oxide (ITO) film (film thickness: 110 nm) having a width of 2 mm had been patterned in a stripe shape was prepared. Subsequently, the substrate was washed with isopropyl alcohol and then subjected to surface treatment by ozone ultraviolet cleaning.

### (Preparation of Vacuum Vapor Deposition)

Each layer was vacuum-deposited by a vacuum vapor deposition method on the surface-treated substrate after washing to form a laminated layer.

First, the glass substrate was introduced into a vacuum vapor deposition tank and pressure inside the tank was reduced to 7.0×10⁻⁵ Pa. Then, each layer was formed in the following order in accordance with the film forming conditions of the respective layers.

### (Production of Electron Transporting Layer 5)

Sublimation purified compound 4,6-bis(3,5-di(pyridin-4-yl)phenyl)-2-methylpyrimidine was deposited to a thickness of 10 nm at a rate of 0.03 nm/sec to produce an electron transporting layer 5.

### (Production of Photoelectric Conversion Layer 4)

N,N-dimethylquinacridone and fullerene C60 were deposited to a film thickness of 125 nm at a ratio of 4:1 (mass ratio) to produce a photoelectric conversion layer 4. The deposition rate was 0.13 nm/sec.

### (Production of Hole Transporting Layer 3)

N,N'-di-1-naphthyl-N,N'-diphenylbenzidine (a-NPD) was deposited to a thickness of 10 nm at a rate of 0.10 nm/second to produce a hole transporting layer 3.

### (Production of Hole Transport Promoting Layer 2)

The compound (A-19) was deposited to a thickness of 10 nm at a rate of 0.20 nm/sec to produce the hole transport promoting layer 2.

### (Production of First Electrode 1)

Finally, a metal mask was disposed so as to be orthogonal to the ITO stripe on the substrate, and thus, a film of the first electrode 1 was formed. Au was deposited to a film thickness of 80 nm on the first electrode. The deposition rate of Au was 0.1 nm/sec.

Thus, a photoelectric conversion element 100 having an area of 4 mm² as shown in FIG. 1 was produced. Current in a dark place (dark current) and external quantum efficiency were evaluated when a voltage of 2.5 V was applied as an absolute value to the photoelectric conversion element produced as described above so that electrons would be transported to the second electrode 6 side and holes would be transported to the first electrode 1 side. The dark current was measured using a source measure unit 2636B manufactured by company Keithley. The external quantum efficiency was measured using a solar cell spectral sensitivity measuring apparatus (manufactured by company Soma Optics). Measurement was carried out with irradiation light at a wavelength of 560 nm and at an intensity of 50 µW/cm². The results are shown in Table 3. The dark current and the external quantum efficiency are relative values with reference values (1.00) and (100) as results in Element Comparative Example 7 described below, respectively. The lower the value of the dark current, the better the performance, and the higher the value of the external quantum efficiency, the better the performance.

### [Element Comparative Example 7]

A photoelectric conversion element of Comparative Example 7 was produced in the same manner as in Element Example 5 except that the photoelectric conversion element did not have the hole transport promoting layer 2, and dark current and external quantum efficiency were measured in the same manner as in Element Example 5. The results are shown in Table 3.

### [Element Comparative Example 8]

A photoelectric conversion element of Element Comparative Example 8 was produced in the same manner as in Element Example 5 except that HAT-CN was used instead of the compound (A-19) used in the production of the hole transport promoting layer 2 of Element Example 5, and dark current and external quantum efficiency were measured in the same manner as in Element Example 5. The results are shown in Table 3.

**[Table 3]**

| | Hole transporting material | Hole transport promoting material | Dark current | External quantum efficiency |
|---|---|---|---|---|
| Comparative Example 7 | NPD | None | 1.00 | 100 |
| Comparative Example 8 | NPD | HAT-CN | 5. 87E-04 | 102 |
| Example 5 | NPD | A-19 | 3. 87E-04 | 104 |

As shown in Table 3, in the elements of the Element Examples using specific photoelectric conversion materials for imaging element, the dark current was suppressed and high external quantum efficiency was obtained as compared with the device of the Element Comparative Example.

### <Evaluation of Film Quality of Hole Transport Promoting Layer> [Reference Example 1]

In Element Example 1, with respect to the laminate in which the hole transport promoting layer was formed on the first electrode, the arithmetic average roughness (nm) of the surface of the hole transport promoting layer was measured using an atomic force microscope (SPM-9600, manufactured by Shimadzu Corporation). The results are shown in FIG. 2 together with an AFM image of the hole transport promoting layer.

### [Comparative Reference Example 1]

A laminate in which HAT-CN was deposited to a thickness of 30 nm on the first electrode instead of the compound (A-19) used in the production of the hole transport promoting layer in Reference Example 1 was produced, and the film quality was evaluated in the same manner as in Reference Example 1. The results are shown in FIG. 2. Note that HAT-CN is a typical hole injection material for organic ELs.

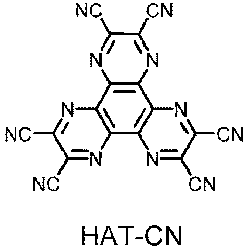

From the results of FIG. 2, it can be understood that the hole transport promoting layer of Reference Example 1 produced using the compound (A-19) had a flat film quality as compared with the hole transport promoting layer of Reference Example 1 produced using HAT-CN. It is considered that the film quality was flat, and thus, adhesion to the adjacent layer was improved in the hole-only device of Element Example 1, resulting in efficient hole transport.

Although the present invention has been described in detail and with reference to specific embodiments, it will be apparent to those skilled in the art that various changes and modifications can be made without departing from the spirit and scope of the invention.

The entire contents of Japanese Patent Application No. 2021-112797, filed on July 7, 2021, are incorporated herein by reference.

### EXPLANATION OF REFERENCE NUMERALS

1 First electrode
2 Hole transport promoting layer
3 Hole transporting layer
4 Light receiving layer (photoelectric conversion layer)
5 Electron transporting layer
6 Second electrode
10 Organic Layer
100 Organic light receiving element

## Claims

1. A hole transport promoting material comprising a compound represented by the following general formula (1) or (2): 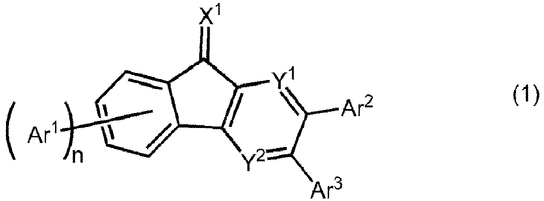 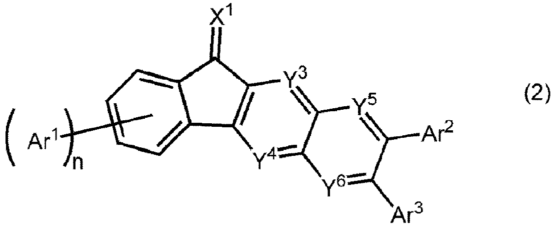 wherein
Y¹, Y², Y³, Y⁴, Y⁵ and Y⁶ each independently represent a nitrogen atom or C-H;
at least one of Y¹ or Y² is a nitrogen atom;
at least one of Y³, Y⁴, Y⁵ or Y⁶ is a nitrogen atom;
Ar¹, Ar² and Ar³ each independently represent:
a hydrogen atom,
a cyano group,
a fluoro group,
a fluoroalkyl group,
an aryl group having 6 to 18 carbon atoms and being optionally substituted with a fluoro group, a fluoroalkyl group, or a cyano group; or
a heteroaryl group having 3 to 17 carbon atoms and being optionally substituted with a fluoro group, a fluoroalkyl group, or a cyano group;
n represents an integer of 1 to 4; and
X¹ is a divalent group represented by the following formula (a) or (b): 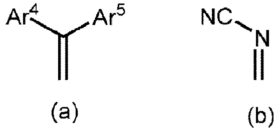 wherein
Ar⁴ and Ar⁵ each independently represent:
a cyano group,
a fluoro group,
a fluoroalkyl group,
an aryl group having 6 to 18 carbon atoms and being optionally substituted with a fluoro group, a fluoroalkyl group, or a cyano group; or
a heteroaryl group having 3 to 17 carbon atoms and being optionally substituted with a fluoro group, a fluoroalkyl group, or a cyano group.

2. The hole transport promoting material according to claim 1, wherein Ar² and Ar³ are cyano groups.

3. The hole transport promoting material according to claim 1 or 2, wherein Ar⁴ and Ar⁵ are cyano groups.

4. A light receiving element material comprising a compound represented by the following general formula (1) or (2): 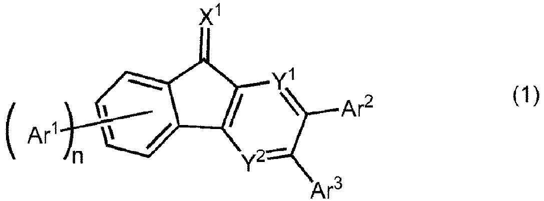 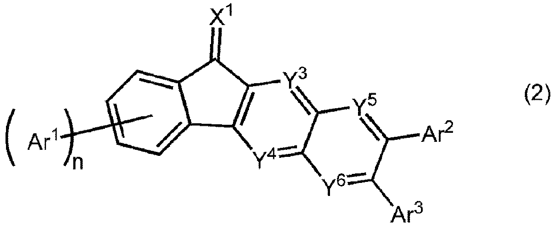 wherein
Y¹, Y², Y³, Y⁴, Y⁵ and Y⁶ each independently represent a nitrogen atom or C-H;
at least one of Y¹ or Y² is a nitrogen atom;
at least one of Y³, Y⁴, Y⁵ or Y⁶ is a nitrogen atom;
Ar¹, Ar² and Ar³ each independently represent:
a hydrogen atom,
a cyano group,
a fluoro group,
a fluoroalkyl group,
an aryl group having 6 to 18 carbon atoms and being optionally substituted with a fluoro group, a fluoroalkyl group, or a cyano group; or
a heteroaryl group having 3 to 17 carbon atoms and being optionally substituted with a fluoro group, a fluoroalkyl group, or a cyano group;
n represents an integer of 1 to 4; and
X¹ is a divalent group represented by the following formula (a) or (b): 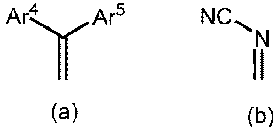 wherein
Ar⁴ and Ar⁵ each independently represent:
a cyano group,
a fluoro group,
a fluoroalkyl group,
an aryl group having 6 to 18 carbon atoms and being optionally substituted with a fluoro group, a fluoroalkyl group, or a cyano group; or
a heteroaryl group having 3 to 17 carbon atoms and being optionally substituted with a fluoro group, a fluoroalkyl group, or a cyano group.

5. The light receiving element material according to claim 4, wherein the light receiving element material is an organic imaging element material for light receiving element.

6. The light receiving element material according to claim 4 or 5, wherein the light receiving element material is a hole transport promoting material for organic imaging element for light receiving element.

7. The light receiving element material according to claim 4 or 5, wherein Ar² and Ar³ are cyano groups.

8. The light receiving element material according to claim 4 or 5, wherein Ar⁴ and Ar⁵ are cyano groups.

9. A cyano compound represented by the following general formula (3), general formula (4), general formula (5) or general formula (6): 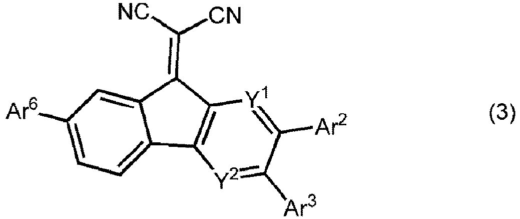 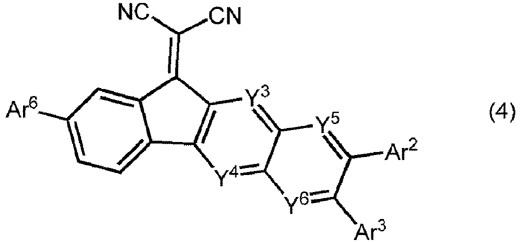 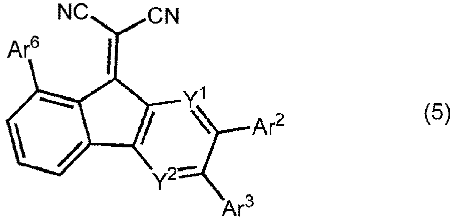 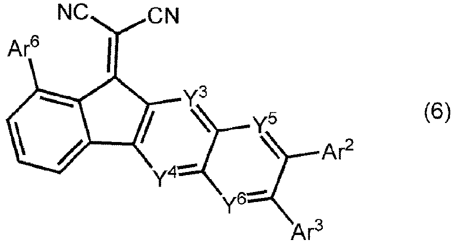 wherein
Y¹, Y², Y³, Y⁴, Y⁵ and Y⁶ each independently represent a nitrogen atom or C-H;
at least one of Y¹ or Y² is a nitrogen atom;
at least one of Y³, Y⁴, Y⁵, or Y⁶ is a nitrogen atom;
Ar⁶ represents:
a cyano group,
a fluoro group, a chloro group, a bromo group, an iodine group,
a fluoroalkyl group,
an aryl group having 6 to 18 carbon atoms and being optionally substituted with a fluoro group, a fluoroalkyl group, or a cyano group; or
a heteroaryl group having 3 to 17 carbon atoms and being optionally substituted with a fluoro group, a fluoroalkyl group, or a cyano group;
provided that when Y⁵ and Y⁶ are C-H, Ar⁶ may be a hydrogen atom, and
Ar² and Ar³ each independently represent:
a hydrogen atom,
a cyano group,
a fluoro group,
a fluoroalkyl group,
an aryl group having 6 to 18 carbon atoms and being optionally substituted with a fluoro group, a fluoroalkyl group, or a cyano group; or
a heteroaryl group having 3 to 17 carbon atoms and being optionally substituted with a fluoro group, a fluoroalkyl group, or a cyano group.

10. The cyano compound according to claim 9, wherein Ar² and Ar³ are cyano groups.

11. An organic light receiving element,
comprising a first electrode and a second electrode, and
having an organic layer between the first electrode and the second electrode,
the organic layer comprising at least a light receiving layer, a hole transporting layer, and a hole transport promoting layer,
the hole transport promoting layer comprising a compound having a partial structure represented by the following formula (Q): 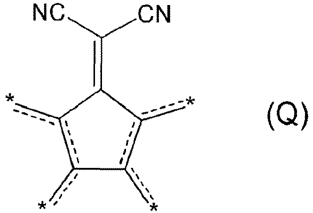 wherein portions in which solid lines and broken lines are parallel each independently represent a single bond or a double bond, and * represents a bond.

12. An organic light receiving element,
comprising a first electrode and a second electrode, and having an organic layer between the first electrode and the second electrode,
the organic layer comprising at least a light receiving layer, a hole transporting layer, and a hole transport promoting layer,
the hole transport promoting layer comprising a compound having a partial structure represented by the following formula (1) or (2): 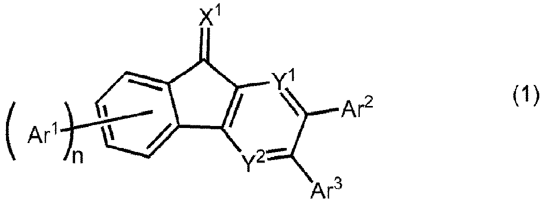 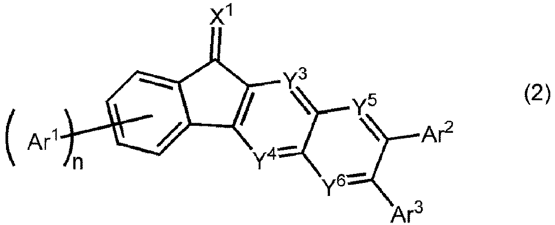 wherein
Y¹, Y², Y³, Y⁴, Y⁵ and Y⁶ each independently represent a nitrogen atom or C-H;
at least one of Y¹ or Y² is a nitrogen atom;
at least one of Y³, Y⁴, Y⁵ or Y⁶ is a nitrogen atom;
Ar¹, Ar² and Ar³ each independently represent:
a hydrogen atom,
a cyano group,
a fluoro group,
a fluoroalkyl group,
an aryl group having 6 to 18 carbon atoms and being optionally substituted with a fluoro group, a fluoroalkyl group, or a cyano group; or
a heteroaryl group having 3 to 17 carbon atoms and being optionally substituted with a fluoro group, a fluoroalkyl group, or a cyano group,
n represents an integer of 1 to 4; and
X¹ is a divalent group represented by the following formula (a) or (b): 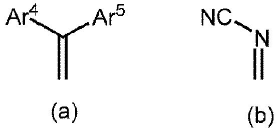 wherein
Ar⁴ and Ar⁵ each independently represent:
a hydrogen atom,
a fluoro group,
a fluoroalkyl group,
an aryl group having 6 to 18 carbon atoms and being optionally substituted with a fluoro group, a fluoroalkyl group, or a cyano group; or
a heteroaryl group having 3 to 17 carbon atoms and being optionally substituted with a fluoro group, a fluoroalkyl group, or a cyano group.

13. The organic light receiving element according to claim 11 or 12, wherein the hole transporting layer and the hole transport promoting layer are adjacent to each other.

14. The organic light receiving element according to claim 11 or 12, wherein the light receiving layer is a layer composed of at least two components.
